Europäisches Patentamt

European Patent Office

Office européen des brevets

(19)

(11) Numéro de publication: **0 369 908**

**A1**

## (12) DEMANDE DE BREVET EUROPEEN

(21) Numéro de dépôt: **89420449.4**

(22) Date de dépôt: **17.11.89**

(51) Int. Cl.5: **A61B 5/11, A61C 19/045**

(30) Priorité: **18.11.88 FR 8815484**

(43) Date de publication de la demande:
**23.05.90 Bulletin 90/21**

(84) Etats contractants désignés:
**AT BE CH DE ES FR GB GR IT LI LU NL SE**

(71) Demandeur: **HENNSON INTERNATIONAL**
**Château de Malissol**
**F-38200 Vienne(FR)**

(72) Inventeur: **Duret, François**
**Rue Paul Claudel**
**F-38690 Le Grand Lemps(FR)**
Inventeur: **Blouin, Jean-Louis**
**Château de Malissol**
**F-38200 Vienne(FR)**

(74) Mandataire: **Maureau, Philippe et al**
**Cabinet GERMAIN & MAUREAU BP 3011**
**F-69392 Lyon Cédex 03(FR)**

(54) **Dispositif de mesure et d'analyse de mouvements du corps humain ou de parties de celui-ci.**

(57) Un dispositif de mesure et d'analyse de mouvements de parties du corps humain, notamment d'une mâchoire, comprend:
- un ensemble de trois diodes électro-luminescentes (12) fixées de façon amovible sur la partie du corps dont le mouvement est à analyser,
- deux capteurs (6) destinés à suivre les mouvements des diodes (12), montés sur un support fixe (4, 5) de façon à ce que les diodes se trouvent dans leur champ,
- et des moyens (24-27) pour commander l'éclairage des diodes, le fonctionnement des capteurs et le traitement des informations fournies par ceux-ci.

FIG.1

EP 0 369 908 A1

# DISPOSITIF DE MESURE ET D'ANALYSE DE MOUVEMENTS DU CORPS HUMAIN OU DE PARTIES DE CELUI-CI

La présente invention a pour objet un dispositif de mesure et d'analyse des mouvements du corps humain par des moyens électroniques et notamment la mesure des mouvements mandibulaires dans le but de permettre la réalisation plus facilement et plus précise de prothèses, le diagnostic rapide des pathologies articulaires et la transmission des informations directement utilisable par une technique de conception et fabrication assistée par ordinateur dans le domaine dentaire, telle que décrite dans le brevet français n° 82 06707.

Les diverses méthodes d'analyse de la cinématique mandibulaire qui ont prévalu jusqu'aux années 1985, étaient basées sur l'utilisation d'instruments mécaniques tous plus ou moins dérivés de mandibulographes dont l'apparition commerciale datait des années 50.

Ces appareils permettaient d'enregistrer une expression relativement restreinte de cette cinématique, puisque limitée aux mouvements dits de bordure de la mandibule. De surcroit l'interprétation des tracés obtenus par ces méthodes a donné lieu à un certain nombre d'erreurs dues à la méconnaissance du cycle fonctionnel et à l'intervention de procédés mécaniques qui entraînaient l'existence d'artéfacts dans les tracés.

Un autre de leur aspect négatif, résidait dans l'encombrement important pour la cavité buccale de procédés de fixation lourds et volumineux avec lesquels il était impossible d'envisager l'enregistrement d'une physiologie normale de ladite cinématique.

L'utilisation d'appareils utilisant un système d'enregistrement peu encombrant dans l'espace buccal et n'intervenant d'aucune manière sur le cycle masticatoire permet aujourd'hui d'enregistrer aisément la cinématique fonctionnelle jusqu'à maintenant écartée de nos préoccupations quotidiennes.

Les travaux de LUNDEEN et GIBBS en avaient donné déjà une première expression avec le "Gnathic réplicator". Ces études ont été largement complétées par de nombreux auteurs qui ont mis en évidence l'aspect plus aléatoire des cycles masticatoires et ont permis une approche plus précise des phénomènes qui interviennent sur l'aspect des cycles : neuro-musculature, qualité des aliments broyés, posture de l'individu etc...

Un certain nombre d'autres appareils ont cherché à représenter le plus clairement possible les données essentiellement caractéristiques de l'appareil manducateur. On ne peut ignorer en particulier les récents travaux de LEWIN qui ont permis la réalisation du "sirognathographe" de SIEMENS, le "Nex K6 diagnostic Système" de Myo-tronics inc, le "LR Candylograph" de Dentron, le "Stereognathograph" du Dr. BURCHHARDT ou le Cyberhoby computer pantograph de DENAR.

Il faut signaler enfin le "visitrainer" décrit pour la première fois en 1981 par l'équipe du Professeur HOBO et du Professeur MORI au Japon. Il utilise une LED (Diode électro-luminescente) fixée sur les incisives mandibulaires. A la même époque, l'équipe du Professeur HOBO fait état d'un système utilisant un dispositif à transfert de charges (CCD) comme moyen de lecture ; le tout relié à différents éléments de calcul (ordinateur) et leurs périphériques associés.

On retrouve, dans ces travaux, les principes développés par BARRIE en 1967 et son "photo-électrique mandibulography". C'est en 1984 qu'apparaît le premier "Saphon visitrainer modèle 1". Ce dernier sera bientôt suivi du modèle C2, puis du modèle 3 (SVT). Ces modèles ont été développés par la firme TOKYO SHIZAISMA C.O. Ltd. et diffèrent surtout par leur approche ergonomique (poids, nombre de points analysés par seconde, etc...).

Le fait de devoir déplacer une caméra unique à trois reprises pour analyser les mouvements dans le plan horizontal, frontal et sagittal empêche de connaître ces déplacements dans l'espace car un individu ne déplace jamais ses articulations de la même manière, ce qui empêche tout raccordement des trois déplacements.

A la même époque JEMT et KARLSON ont montré que l'utilisation de deux caméras et une diode fixée sur les incisives pouvaient permettre l'analyse des mouvements mandibulaires en trois dimensions. En réalité, ceci n'est pas exact. En particulier, on se rend compte qu'un mouvement de rotation pur, existant souvent dans le corps humain, ne peut pas être détecté correctement.

La présente invention a pour but de remédier à ces inconvénients en fournissant des analyses en trois dimensions, adaptées à toutes applications odontologiques et médicales, en précisant les moyens permet tant l'analyse des mouvements et en fournissant un signal directement analysable par un ordinateur, ceci d'une manière très rapide tout en offrant la possibilité de vérification de l'analyse, sans aucune action manuelle intermédiaire comme le constituait jusqu'à présent le déplacement des caméras.

Les moyens mis en oeuvre sont économiquement intéressants dans la mesure où ils s'appliquent à un large marché et qu'ils apportent un gain effectif de temps et de main d'oeuvre.

A cet effet, le dispositif qu'elle concerne comprend :
- un ensemble de trois diodes électro-luminescentes fixées de façon amovible sur la partie du corps dont le mouvement est à analyser,
- deux capteurs destinés à suivre les mouvements des diodes, montés sur un support fixe de façon à ce que les diodes se trouvent dans leur champ,
- et des moyens pour commander l'éclairage des diodes, le fonctionnement des capteurs et le traitement des informations fournies par ceux-ci.

Avantageusement, les diodes électro-luminescentes émettent dans l'infra-rouge, et possèdent un angle d'émission de l'ordre de 180°. Les diodes électro-luminescentes émettent à une longueur d'onde de 900 mm, avec une puissance de 30 mW environ. Dans le cas d'une détection des mouvements d'une mâchoire, les diodes, fixées par exemple sur les incisives, présentent l'intérêt, du fait qu'elles émettent dans l'infrarouge, de disposer d'une certaine immunité par rapport à l'éclairage ambiant, et de permettre une détection lèvres fermées.

En outre, l'angle d'émission de 180° permet le suivi des mouvements simultanément au niveau des deux capteurs. La sensibilité des capteurs se situe aux environs de 900 mm. La fréquence d'échantillonnage globale étant de 1 à 2 KHz sur une durée d'enregistrement de 20 secondes, la résolution obtenue est sensiblement supérieure à celle observée avec les autres appareils.

Ce dispositif possède des moyens de synchronisation assurant l'éclairage successif des trois diodes pendant de brefs intervalles de temps, et la mise en fonctionnement simultané des capteurs.

En outre, ce dispositif comprend deux étages de mise en forme du signal permettant de préparer l'information et de la transmettre pour la numérisation, un étage de numérisation assurant la conversion analogique-numérique du signal, sous le contrôle des moyens de synchronisation, et un étage de stockage permettant de stocker les informations et de les visualiser sur un moniteur graphique. Il est à noter qu'un bus de communication externe permet de transmettre l'ensemble des informations au système de CFAO.

Avantageusement, les trois diodes électro-luminescentes sont disposées aux trois sommets d'un triangle. Le positionnement de ses trois diodes permet de répondre aux exigences requises pour l'enregistrement simultané du mouvement d'un point de la mandibule par rapport à trois plans qui sont utilisés couramment en dentisterie à savoir le plan horizontal ou plan d'occlusion, le plan frontal et le plan sagittal.

De toute façon l'invention sera bien comprise à l'aide de la description qui suit, en référence au dessin schématique annexé, représentant, à titre d'exemples non limitatifs, plusieurs formes d'exécution de ce dispositif :

Figure 1 est une vue en perspective d'un premier dispositif de saisie des images ;

Figure 2 est une vue en perspective et à échelle agrandie d'une partie d'arcade dentaire et du dispositif de fixation des diodes sur celle-ci ;

Figure 3 est une vue en perspective d'un dispositif de mesure de la distance entre les capteurs et les diodes ;

Figure 4 est une vue très schématique montrant la mise en oeuvre du dispositif de figure 3 ;

Figures 5 et 6 sont deux vues correspondant aux figures 3 et 4 respectivement, montrant le dispositif de calcul de la distance entre les diodes et le récepteur, dans une seconde forme d'exécution ;

Figure 7 est une vue d'une carte de traitement des signaux ;

Figure 8 est un schéma synoptique de l'installation ;

Figure 9 représente le trajet optique de la mesure ;

Figure 10 est une vue très schématique en perspective de l'ensemble d'une installation ;

Figure 11 est une vue d'une variante du dispositif support des récepteurs ;

Figure 12 est une vue en perspective d'un autre dispositif support des récepteurs ;

Figure 13 est une vue en perspective d'une fourchette de saisie d'empreinte destinée à être utilisée en relation avec le dispositif de figure 12 ;

Figure 14 est une vue partielle de côté de la fourchette de figure 13 ;

Figure 15 est une vue partielle de dessus de la fourchette de figure 13 ;

Figure 16 est une vue en perspective d'un équipement complémentaire du dispositif de figure 12 ;

Figure 17 est une vue de côté d'un articulateur.

Les différents dispositifs, représentés au dessin, sont destinés à la mesure du déplacement et de l'analyse des mouvements d'une mâchoire. Le patient est équipé d'un casque 2 muni de moyens de réglage 3 de sa taille, sur la face avant duquel sont fixés deux bras 4 sensiblement horizontaux et divergents, à l'extrémité libre de chacun desquels est montée une tige 5 sensiblement verticale équipée, à son extrémité inférieure, d'un capteur infrarouge 6 formant caméra. La position de chaque capteur 6 peut être réglée tant dans la direction du bras 4 que dans la direction de la tige 5 correspondante par l'intermédiaire de vis sans fin, respectivement 7, 8. Les bras 4 sont positionnés pour que les caméras fassent entre elles un angle compris entre 35 et 120°, et de préférence 90°.

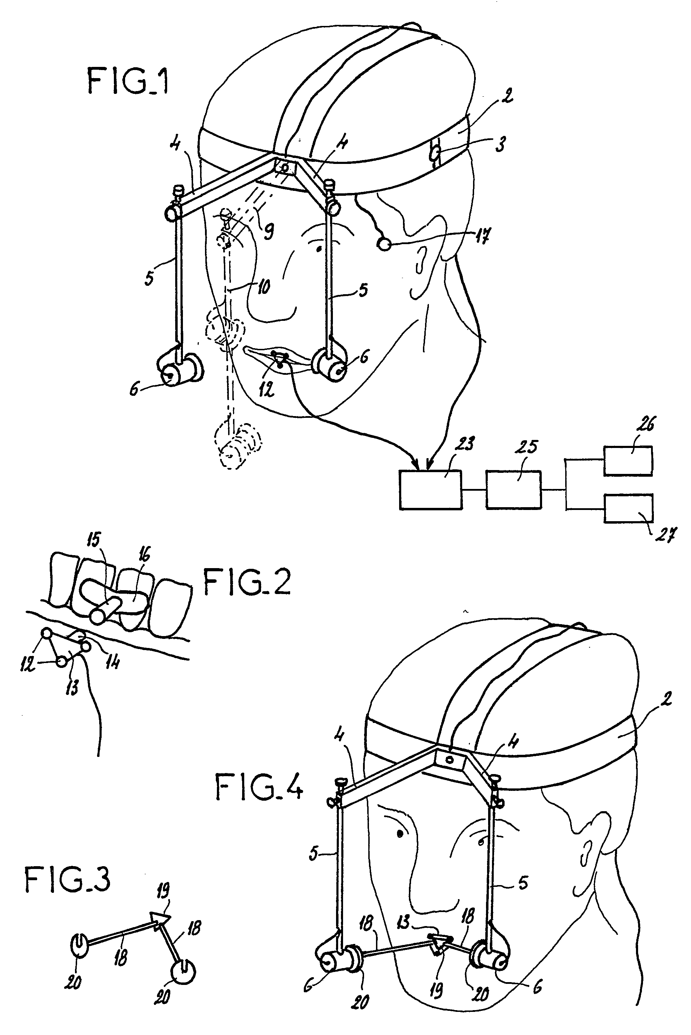

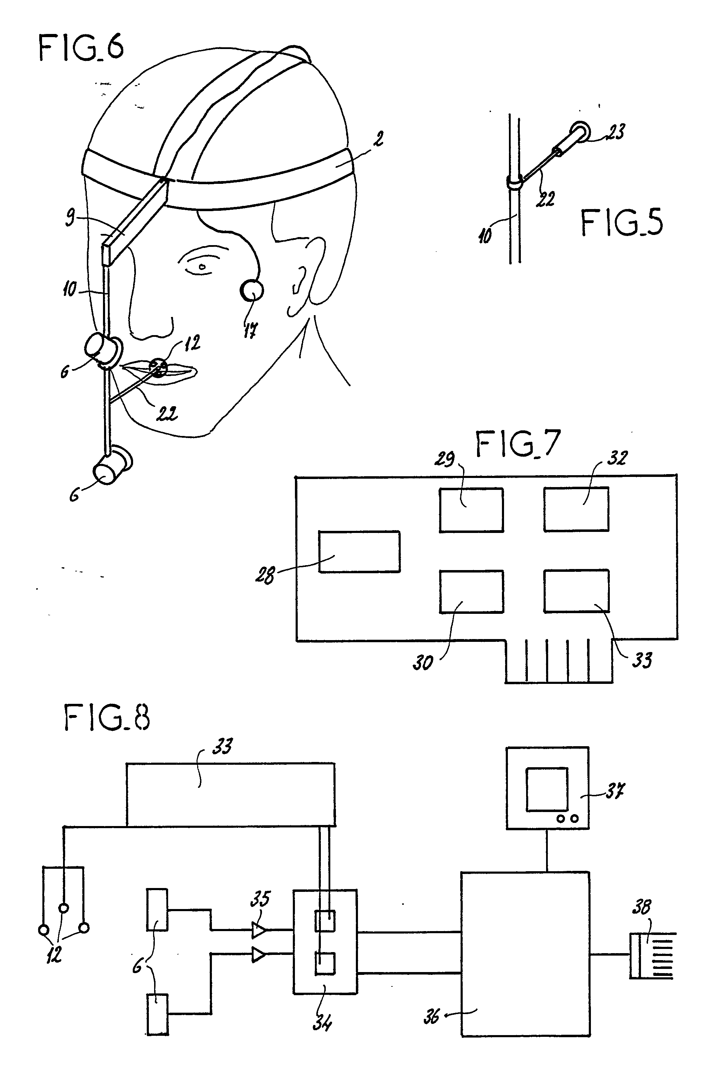

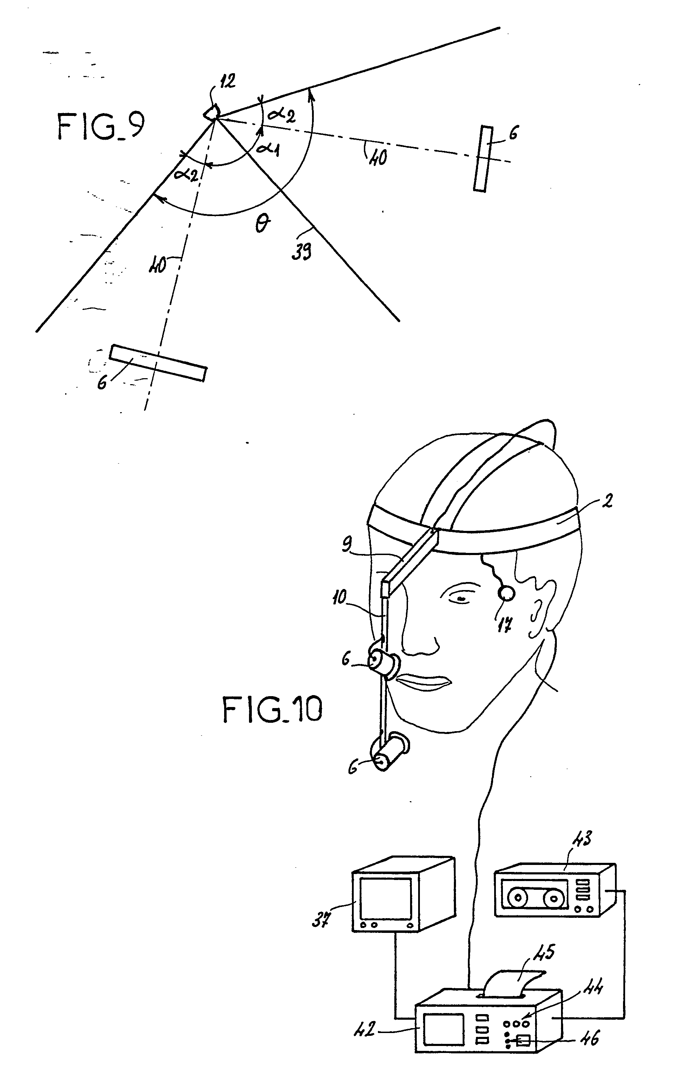

Dans une variante, représentée en traits mixtes à la figure 1, ainsi qu'aux figures 6 et 10, les deux capteurs 6 sont portés par un bras 9 et une tige 10 uniques, ce qui simplifie les réglages et limite l'encombrement.

Ce dispositif comprend trois diodes électroluminescentes 12 émettant dans l'infrarouge, qui sont fixées sur un même support 13, et disposées aux trois sommets d'un triangle. Ce support 13 est très rigide afin d'éviter un mouvement relatif des diodes 12 au cours du déplacement de l'organe sur lequel le support est fixé. Ce support 13 est solidaire d'une tige 14 destinée à être engagée dans un élément tubulaire 15 solidaire d'une platine support 16 destinée à être fixée de façon amovible sur les dents. Cette fixation est réalisée à l'aide d'un ciment photo-polymérisable. La fixation des diodes sur les dents est réalisée en deux temps : montage du support 15, 16 sur les dents puis, fixation sur cet ensemble du support de diodes avec stabilisation par coincement, par aimant ou encore par un effet ressort.

Un capteur sonore 17 permet de connaître le moment où les dents entrent en contact et de séparer chaque cycle comportant une mastication.

Afin d'avoir un rapport exact entre les déplacements et leur représentation, avant d'effectuer les mesures, il est procédé à la mise en place d'une règle graduée entre les diodes 12 et les caméras avant de fixer définitivement la position de celles-ci. Le réglage de la position de caméras est effectué en jouant sur les vis 7, 8.

Dans la forme d'exécution représentée à la figure 3, le dispositif d'étalonnage de la distance comprend deux tiges 18 reliées l'une à l'autre par une plaquette 19 destinée à venir en appui contre les diodes, et équipée chacune à son autre extrémité d'une plaquette 20 destinée à venir prendre appui contre la caméra. La mise en oeuvre de cet étalonnage est schéma tisée à la figure 4. Les figures 5 et 6 représentent le mode d'étalonnage dans le cas où les deux capteurs 6 sont portés par un seul bras. Dans ce cas, il n'est prévu qu'une seule tige 22 montée, par exemple, coulissante sur la tige verticale 10 et équipée, à son extrémité libre, d'une plaquette d'appui unique 23.

Les informations fournies par les caméras sont transmises à une unité électronique 23, traitées dans une unité d'analyse 25 avec possibilité de connexion, soit dans le cadre d'une commande assistée par ordinateur 26, soit d'une imagerie classique 27.

L'ensemble caméras et diodes est sous la dépendance de plusieurs étages électroniques et informatiques qui sont :
- Un étage de mise en forme du signal permettant la sommation dans les deux plans des caméras de la succession des points d'analyse. En effet, il importe d'avoir la forme globale de la courbe, et non le suivi d'un point.

Cet étage fonctionne comme suit :
. réception du signal,
. conversion courant/tension;
. entrée de la tension sur quatre entrées différentielles par diodes,
. conversion analogique-digitale,
. entrée sur un calculateur et, éventuellement, une liaison avec un système de CAO.
- Un étage de calculateur permettant, grâce à un logiciel approprié de transformer les courbes analysées sur les deux plans de la caméra en projection sur les trois plans du visage utilisés en dentisterie, à savoir le plan horizontal ou plan d'occlusion, le plan frontal et le plan sagittal. La carte électronique utilisée est représentée à la figure 7, et comprend une mémoire 28, une interface conversion/mémoire 29, une interface unité centrale/mémoire 30, un système de conversion 32, et un système 33 de synchronisation des signaux.

En dehors des cartes dont les fonctions ont déjà été décrites, il faut noter la présence d'une limite mémoire permettant de stocker les informations arrivant sous forme de points, afin de construire les courbes et surtout la synchronisation permettant d'éclairer, successivement, chaque diode 12 et d'indiquer, au niveau de chaque capteur 6 quelle diode émet au moment de la réception du point lumineux.

Il est ainsi possible de recevoir les trajets respectifs des trois points sur chaque capteur 6. C'est à partir de ces six courbes que sont reconstituées les trois courbes spécifiques du mouvement rapportées ensuite sur les trois plans dentaires connus.

La figure 8 représente l'ensemble du système dans lequel les différents éléments sont désignés par les références suivantes :

Les diodes 12 sont sous la commande du système 33 de synchronisation. Ce système est également relié à l'étage de numérisation 34 auquel sont connectés les deux capteurs 6 par l'intermédiaire d'un étage de mise en forme 35. L'étage de numérisation est relié à l'unité centrale 36 sur laquelle sont connectés un écran de visualisation 37 et un étage de transmission désigné par la référence générale 38.

Comme le montre la figure 9, l'axe principal d'émission d'une diode 6 possède un angle d'émission $\theta$ égal $\alpha 1 + 2 \alpha 2$ où $\alpha 1$ est l'angle formé par les axes optiques orthogonaux 40 des deux capteurs 6 et où $2 \alpha 2$ est l'angle d'ouverture angulaire des optiques des capteurs.

Les deux capteurs définissent chacun un repère plan, les deux repères ainsi obtenus formant avec le plan des diodes un trièdre permettant de repérer chaque diode émettrice dans l'espace. Si ce trièdre est un trièdre droit, les calculs ultérieurs de corrélation sont éliminés. Si une diode émettrice définit les mouvements, à l'exception des rotation centrées, deux émetteurs ne peuvent détecter les rotation autour de l'axe de chaque émetteur alors que la mise en oeuvre de trois émetteurs permet la détection de tous les mouvements de translation et de rotation.

A titre d'exemples, les valeurs suivantes peuvent être utilisées :
- durée de l'enregistrement 20 s,
- fréquence d'échantillonnage globale 1KHz,
- nombre d'échantillons globaux 20 000,
- nombre d'échantillons secondaires (3 par échantillon global)60 000,
- quatre coordonnés par échantillon secondaire 240 000,
- nombre d'octets par coordonné : 2,
- nombre total d'octects : 450 000.

Il est obtenu entre 2 000 et 4 000 valeurs par cycle de 1 seconde, ce qui représente une précision de 10 à 20 fois supérieure à celle d'appareils connus.

En pratique, le casque 2 est placé sur la tête du patient avant fixation des capteurs 6 et étalonnage, grâce aux dispositifs de réglage 7, 8. L'ensemble est connecté sur le corps d'un système informatique renfermant la carte d'analyse et comportant notamment un oscilloscope 42, un moniteur 37 et, éventuellement, un magnétoscope 43. Les diodes 12 sont éclairées alternativement et leur signal est perçu par les capteurs 6. Au début de l'analyse, la mâchoire du patient est en position d'occlusion serrée. L'appareil est mis en route après quoi il est demandé au patient d'effectuer un ensemble de mouvements qu'il est possible de visualiser soit de façon globale, soit au niveau des différents plans sur lesquels l'image est projetée par sélection, grâce aux différents boutons 44. Ces informations peuvent être simplement visualisées ou éditées sur un papier 45.

Par ailleurs, certaines informations accessoires, telles que calcul d'angle, amplitude du mouvement, temps du cycle, peuvent être proposées par sélection à l'aide de boutons 46.

Une connection directe avec un dispositif de CFAO (conception et fabrication assistées par ordinateur) peut être utilisé, comme décrit notamment dans le brevet français 82 06707. Une prothèse dentaire peut être construite par empreinte optique, création de la maquette en surface mathématique en fonction de cette empreinte et usinage direct. Le fait de ne pas passer par une maquette en cire oblige à travailler sur des surfaces gauches à l'écran, ce qui interdit l'utilisation d'articulateurs dentai res traditionnels.

La présente invention permet, selon un autre de ses aspects, de transcrire sur les surfaces modélisées en statique le mouvement dynam ique, lorsque le patient déplace ses membres ou sa mâchoire. Cela permet, d'une part, de changer certains angles ou surfaces qui pourraient interférer, mais aussi de développer des équations de surfaces intégrant la notion du temps.

Pour accéder à une information sur les plans séparément ou ensemble, sans connaître le développement dans l'espace et en négligeant les éventuelles rotations pures, le système, selon l'invention, offre la possibilité dans son circuit électronique de pouvoir ne suivre qu'une seule diode. Cette analyse est assurée par une construction parfaitement parallèle des composants et circuits et par un accès au système d'horloge.

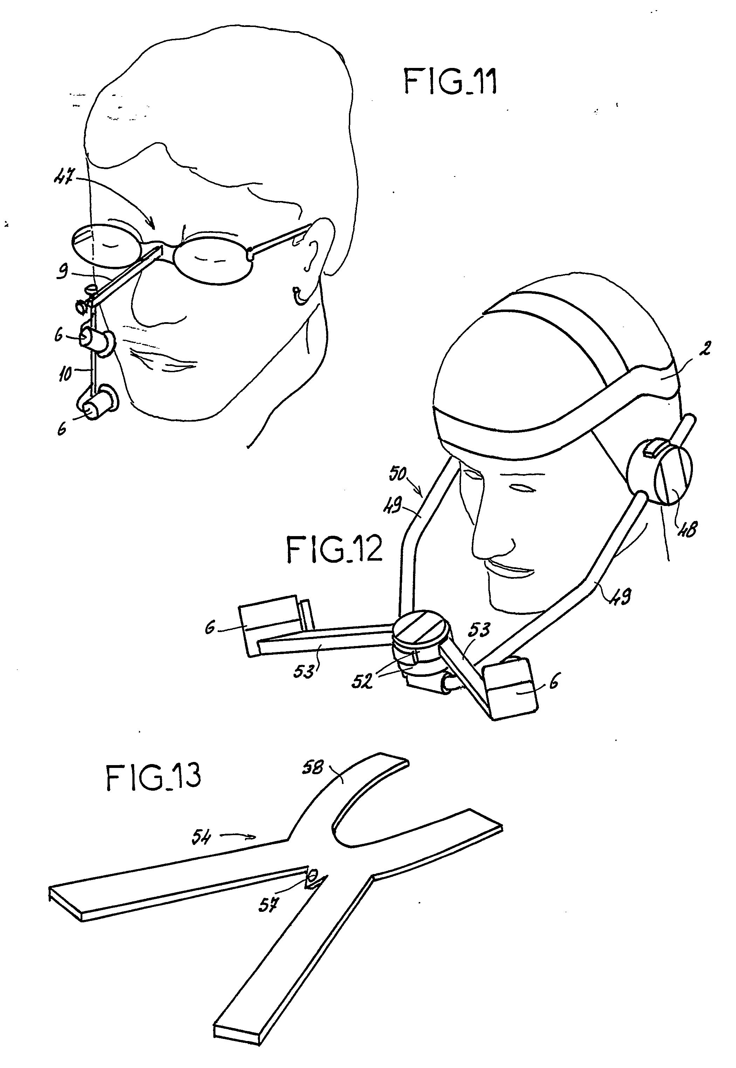

La figure 11 représente une variante d'exécution du dispositif support de capteurs décrit précédemment, dans lequel les mêmes éléments sont désignés par les mêmes références. Dans ce cas, le système au lieu d'être constitué par un casque est constitué par des lunettes réglables 47 portées par le patient.

La figure 12 représente encore un autre dispositif support des capteurs. Dans ce dispositif, le casque 2 est équipé de deux noix 48 disposées au niveau des oreilles du patient, et permettant un réglage en coulissement et en rotation des deux branches 49 d'un arceau 50 dont la partie centrale, disposée en regard du visage du patient, est équipée de deux noix 52 destinées chacune au montage avec réglage en coulissement et en translation, d'une tige 53 à l'extrémité libre de laquelle est monté un capteur 6.

Les moyens de blocage au niveau des noix 48 et 52 peuvent être purement mécaniques ou encore pneumatiques ou hydrauliques, ce qui permet de laisser les éléments totalement libres jusqu'à l'instant de leur blocage. Afin de permettre l'exploitation des informations obtenues sur un articulateur mécanique traditionnel, le dispositif selon l'invention comprend également une fourchette spéciale 54 sur laquelle le patient serrera les dents, cette fourchette permettant non seulement de positionner les modèles de plâtre 55 sur un articulateur 56, tel que celui représenté à la figure 17, mais encore de réaliser la fixation des diodes sur les dents du patient.

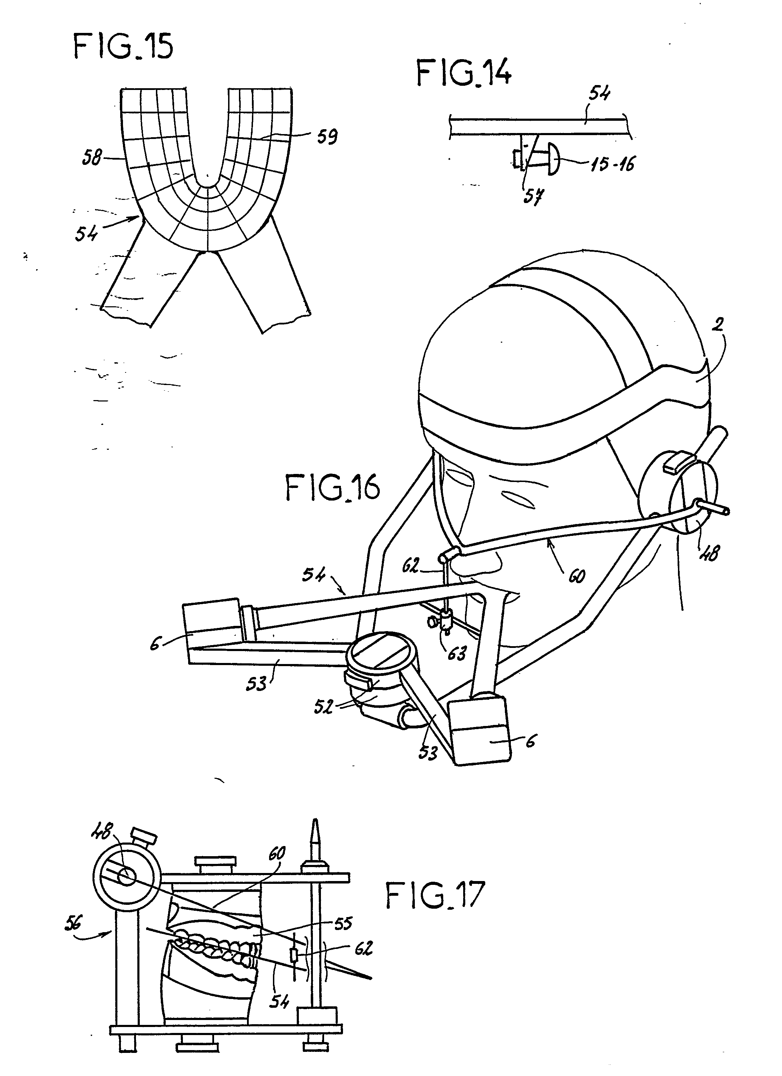

A cet effet, la fourchette 54 comporte, comme montré à la figure 14, une cavité 57 dans laquelle est destinée à être engagé le dispositif 15, 16 de fixation des diodes sur les dents. La plaque 58 de la fourchette étant revêtue, sur ses deux faces, d'une pâte dure d'empreinte, la fourchette est introduite dans la bouche du patient.

Le patient serre la plaque entre ses dents et marque l'empreinte de ses dents sur la pâte, la fixation du support de diodes 15, 16 se faisant au même moment, ainsi que le réglage de la position des capteurs 6. A cet instant, il est possible de connaître la position des caméras, des diodes et des empreintes de dents par rapport à l'axe de rotation de mâchoires qui correspond à l'axe passant par les noix 48.

Pour modifier les coordonnées occlusales d'une surface modélisée, il est nécessaire de connaître la position de la future prothèse par rapport aux diodes dans le cas de l'application du dispositif à la réalisation d'une prothèse.

A cet effet, comme montré à la figure 15, la plaque 58 de la fourchette 54 peut être réalisée en un matériau transparent et comporter un système de repère 59 obtenu par marquage ou par gravure.

Par ailleurs, il est possible, grâce au dispositif selon l'invention de connaître la hauteur exacte et la position du plan de Francfort, plan passant par la bouche au niveau de la base du nez et par l'axe des oreilles, par rapport à la fourchette 54.

A cet effet, le dispositif comprend un arceau 60 fixé sur les deux noix 48, dont les branches sont orientées parallèlement au plan de référence facial et dont la partie centrale est équipée d'une tige 62 sensiblement verticale comportant un organe de mesure 63 de la position de la fourchette 54 par rapport à ce plan.

La connaissance de la position de ces points, fournie par le dispositif selon l'invention qui est utilisé alors comme articulateur électronique, permet de positionner les modèles de plâtre des empreintes du patient sur la fourchette, de mettre l'ensemble de la fourchette et des empreintes dans un articulateur 56 traditionnel selon les méthodes classiques connues à ce jour et de régler les valeurs de cet articulateur (angle de Benett, pente condyliène, angle cuspidien...) en tenant compte des valeurs directement fournies par le dispositif selon l'invention. Cette technique permet de bénéficier, pour le réglage, de l'articulateur de mesure effectuée de façon très précise par électronique.

Comme il ressort de ce qui précède, l'invention apporte une grande amélioration à la technique existante en fournissant un dispositif de conception simple, de fonctionnement très fiable offrant de larges possibilités d'utilisation, tout en étant très aisé à mettre en oeuvre par le praticien.

**Revendications**

1. - Dispositif de mesure et d'analyse de mouvements du corps humain ou de parties de celui-ci, caractérisé en ce qu'il comprend :
- un ensemble de trois diodes électro-luminescentes (12) fixées de façon amovible sur la partie du corps dont le mouvement est analyser,
- deux capteurs (6) destinés à suivre les mouvements des diodes (12), montés sur un support fixé (4, 5) de façon à ce que les diodes se trouvent dans leur champ,
- et des moyens (24-27) pour commander l'éclairage des diodes, le fonctionnement des capteurs et le traitement des informations fournies par ceux-ci.

2. - Dispositif selon la revendication 1, caractérisé en ce que les diodes électro-luminescentes (12) émettent dans l'infra-rouge, et possèdent un angle d'émission de l'ordre de 180°.

3. - Dispositif selon l'une quelconque des revendications 1 et 2, caractérisé en ce qu'il possède des moyens de synchronisation (33) assurant l'éclairage successif des trois diodes (12) pendant de brefs intervalles de temps, et la mise en fonctionnement simultané des capteurs (6).

4.- Dispositif selon l'une quelconque des revendications 1 à 3, caractérisé en ce qu'il comprend deux étages (35) de mise en forme du signal permettant de préparer l'information et de la transmettre pour la numérisation, un étage (34) de numérisation assurant la conversion analogique-numérique du signal, sous le contrôle des moyens de synchronisation (33), et un étage de stockage (36) permettant de stocker les informations et de les visualiser sur un moniteur graphique (37).

5. - Dispositif selon l'une quelconque des revendications 1 à 4, caractérisé en ce que les trois diodes électro-luminescentes (12) sont disposées aux trois sommets d'un triangle.

6.- Dispositif selon l'une quelconque des revendications 1 à 5, caractérisé en ce que l'angle formé par les axes des deux capteurs (6) sont compris entre 35° et 120°.

7. - Dispositif selon la revendication 6, caractérisé en ce que l'angle formé par les axes des deux capteurs (6) est de 90°.

8.- Dispositif selon l'une quelconque des revendications 1 à 7, caractérisé en ce que les trois diodes électro-luminescentes (12) sont montées de façon définitive sur un même support rigide (13) destiné à être fixé de façon amovible sur la partie de corps dont l'analyse du mouvement doit être effectuée.

9. - Dispositif selon la revendication 4, caractérisé en ce que, dans le cas de la mesure et de l'analyse des mouvements mandibulaires, les moyens de traitement de l'information comprennent un étage permettant de transformer les courbes analysées sur les deux plans des capteurs en projection sur les trois plans utilisés en dentisterie : le plan horizontal ou plan d'occlusion, le plan frontal et le plan sagittal.

10. - Dispositif selon la revendication 8, caractérisé en ce que, dans le cas de la mesure et l'analyse des mouvements mandibulaires, il comprend un premier support (15, 16)destiné à être fixé de façon amovible sur une dent, tel que par un ciment photo-polymérisable, et à recevoir le support rigide (13) portant les diodes (12).

11. - Dispositif selon l'une quelconque des revendications 1 à 10, caractérisé en ce que, dans le cas de la mesure et de l'analyse des mouvements mandibulaires, il comprend un casque (2) équipé d'au moins un bras (4) portant les deux capteurs (6), avec possibilité de réglage de la distance et de l'inclinaison de ceux-ci par rapport aux diodes.

12. - Dispositif selon la revendication 11, caractérisé en ce que le casque est équipé de deux noix (48) qui, disposées au niveau des oreilles du patient, permettent un réglage en coulissement et en rotation des deux branches (49) d'un arceau (50) dont la partie centrale est disposée en regard du visage du patient, cette partie centrale étant équipée de deux noix (52) d'axes orthogonaux aux premières noix citées, destinées chacune au montage avec réglage en coulissement et en rotation d'une tige (53) à l'extrémité libre de laquelle est monté un capteur (6).

13. - Dispositif selon l'une quelconque des revendications 11 et 12, caractérisé en ce que les moyens (7, 8) de réglage et de blocage des tiges support de capteur sont mécaniques.

14. - Dispositif selon l'une quelconque des revendications 11 et 12, caractérisé en ce que les moyens de blocage des tiges support de capteur sont hydrauliques.

15. - Dispositif selon l'une quelconque des revendications 11 à 14, caractérisé en ce qu'il comprend un capteur sonore (17) permettant de déterminer le moment où les dents du patient entrent en contact.

16. - Dispositif selon l'une quelconque des revendications 11 à 15, caractérisé en ce qu'il comporte une fourchette (54) dont une extrémité (58) en forme de plaque est revêtue, sur ses deux faces, de pâte dure d'empreinte, et qui est équipée, sur l'une de ses faces et en retrait de la plaque, d'une cavité (57) portant le support (15, 16) de diodes (12), cette fourchette étant destinée à être introduite dans la bouche du patient pour réaliser la fixation des diodes et la prise d'empreinte, tandis qu'il est procédé au réglage des capteurs (6).

17. - Dispositif selon la revendication 16, caractérisé en ce que la fourchette (54) est réalisée en un matériau transparent et comporte un système de repérage (59).

18. - Dispositif selon l'ensemble des revendications 12 et 16, caractérisé en ce que les deux noix (48), situées au niveau des oreilles du patient, sont équipées d'un arceau (60) dont les branches sont orientées parallèlement au plan de référence facial et dont la partie centrale est équipée d'une tige verticale (62) équipée d'un organe (63) de mesure de la distance entre l'arceau et la fourchette (54).

FIG.1

FIG.2

FIG.3

FIG.4

FIG.6

FIG.5

FIG.7

FIG.8

FIG.9

FIG.10

FIG.11

FIG.12

FIG.13

FIG.15

58

59

54

FIG.14

54

15-16

57

FIG.16

2

54

62

60

48

6

53

63

52

53

6

48

60

FIG.17

56

55

62

54

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| Y | DE-A-3312262 (K.K. MORITA SEISAKUSHO)<br>* abrégé *<br>* page 6, ligne 16 - page 8, ligne 13 *<br>* page 13, ligne 29 - page 14, ligne 6 *<br>* page 14, lignes 17 - 23; figures 1, 6 * | 1, 4-6, 8 | A61B5/11<br>A61C19/045 |
| A | | 9, 11 | |
| Y | EP-A-263316 (S. NEUMEYER)<br>* abrégé *<br>* colonne 4, ligne 3 - colonne 7, ligne 7 *<br>* colonne 11, lignes 27 - 46 *<br>* colonne 12, ligne 54 - colonne 13, ligne 9; figures 1, 3, 7 * | 1, 4-6, 8 | |
| A | | 2, 10 | |
| A | EP-A-188819 (M. HANSEN)<br>* page 7, ligne 4 - page 8, ligne 16 *<br>* page 8, lignes 26 - 34; figures 1, 3 * | 1, 3-5, 10, 11 | |
| A | EP-A-4888 (SIEMENS A.G.)<br>* abrégé *<br>* page 5, lignes 8 - 19; figure 1 * | 1, 11, 13 | **DOMAINES TECHNIQUES RECHERCHES (Int. Cl.5)** |
| A | EP-A-210668 (J. PUSCHMANN)<br>* abrégé * | 16, 17 | A61B<br>A61C |

Le présent rapport a été établi pour toutes les revendications

| Lieu de la recherche | Date d'achèvement de la recherche | Examinateur |
|---|---|---|
| LA HAYE | 14 FEVRIER 1990 | FERRIGNO A. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
                             
& : membre de la même famille, document correspondant

EPO FORM 1503 03.82 (P0402)